Europäisches Patentamt

(19) European Patent Office

Office européen des brevets

(11) Publication number : 0 376 000 B1

(12) EUROPEAN PATENT SPECIFICATION

(45) Date of publication of patent specification :
30.10.91 Bulletin 91/44

(51) Int. Cl.⁵ : **B01J 8/04**, C01C 1/04,
C07C 29/15

(21) Application number : 89122322.4

(22) Date of filing : 04.12.89

(54) Reactor for exothermic heterogeneous catalytic synthesis.

(30) Priority : 09.12.88 CH 4551/88

(43) Date of publication of application :
04.07.90 Bulletin 90/27

(45) Publication of the grant of the patent :
30.10.91 Bulletin 91/44

(84) Designated Contracting States :
AT DE ES FR GB GR IT NL

(56) References cited :
EP-A- 0 297 474
EP-A- 0 332 757
US-A- 4 755 362

(73) Proprietor : AMMONIA CASALE S.A.
Via della Posta 4
CH-6900 Lugano (CH)
Proprietor : Zardi, Umberto
Via Lucino 57
CH-6932 Breganzona (TI) (CH)

(72) Inventor : Zardi, Umberto
Via Lucino 57
CH-6932 Breganzona (CH)
Inventor : Pagani, Giorgio
Salita dei Frati 13
CH-6900 Lugano (CH)

(74) Representative : Incollingo, Italo
AMMONIA CASALE S.A. Via della Posta 4
CH-6900 Lugano (CH)

## Description

This invention concerns a reactor for exothermic heterogeneous catalytic synthesis under pressure, and more particularly for the catalytic synthesis of ammonia, methanol, etc., consisting of an outer shell, of an internal cartridge, of several catalytic beds formed by baskets consisting of coaxial cylindrical walls enclosing a granular catalyst, said baskets comprising two baskets at the upper and lower ends and at least an intermediate basket, and of the means to adjust and transfer reaction heat.

Reactors of the above-mentioned type are described in numerous patents by the Applicants. More particularly, in base Patent US No. 4,372,920 the description is given for the first time of a reactor which is today widely known and referred to with the expression "axial-radial" ; in US Patent No. 4,405,562 the Applicants have described a particular type of heat exchanger for axial-radial catalytic beds, while the subject of their most recent US Patent No. 4,755,362 consists of a system for reducing energy consumption in classic axial-type reactors by modifying them in situ so as to turn them into axial-radial reactors.

In a recent patent application the Applicants have described a reactor with quenching, with axial-radial gas flow in the catalytic beds where the baskets are independent of the cartridge shell. The preferred configuration of this type of reactor is with three adiabatic beds with two intermediate quenches and with a gas/gas exchanger placed at the bottom, the first two catalytic beds being run through by the gas flowing centrifugally while the gas running through the third bed flows centripetally.

With this preferred embodiment it is possible to dispense with the necessity of providing those flanged connections between the various beds which are indispensable when the baskets are integral with the cartridge shell.

In a further patent application a particular solution is described, in which the bottoms of the baskets are inverted, permitting a better utilization of the available volume, and in which the gas flows outwardly in the first basket and inwardly in the other two. The configuration is with three beds with a quench, an interchanger and possibly a base exchanger.

Continuing their research, the Applicants have now found that it is possible to transfer the characteristics peculiar to this solution to quench-type reactors (without interchanger) with appreciable advantages both in construction and in operation, thus achieving greater structural simplicity, better operation and smaller costs.

These and other advantages are obtained with a reactor as described in the introduction to the main claim and to the description, which is now characterized by the fact that :

— the cartridge-forming shell is substantially independent of the catalytic baskets ;

— at least a first end basket rests on an intermediate basket which in turn rests on a packing ring integral with said cartridge shell, the bottoms of said two coupled basket being inversely curved with respect to the curve at the bottom of the reactor ;

— in said first end bed a collector is placed between the basket's external wall and the inside face of the cartridge shell ;

— in said intermediate bed a quench collector is placed inside the internal cylindrical wall forming a basket ; and

— a gas/gas heat exchanger is placed in a central position inside the cylindrical wall of at least the catalytic basket closest to the reactor outlet for hot reacted gas.

In a first embodiment of the invention, the reactor comprises three beds, the upper bed has the quench collector on its outer periphery with centripetal flow of the quench gas, the intermediate bed resting on the packing ring and acting as support for said first upper basket has the quench collector inside its internal wall with the quench gas flowing centrifugally, the third bed has running through its centre the gas/gas exchanger, the synthesis and quench gas being fed from the top while the reacted gas leaves from the bottom.

In a second embodiment of the invention the upper bed with quench collector on its outer periphery, as well as the intermediate basket with quench collector inside its internal wall, are both run through the centre by a gas/gas exchanger, the synthesis gas being fed from the bottom, the quench gas being fed from the top, and the reacted gas outlet being placed at the top.

The various aspects and advantages of the invention will be better illustrated by the following description of preferred but not limitative embodiment, as shown in the attached drawings which are two schematic and partial views in cross-section.

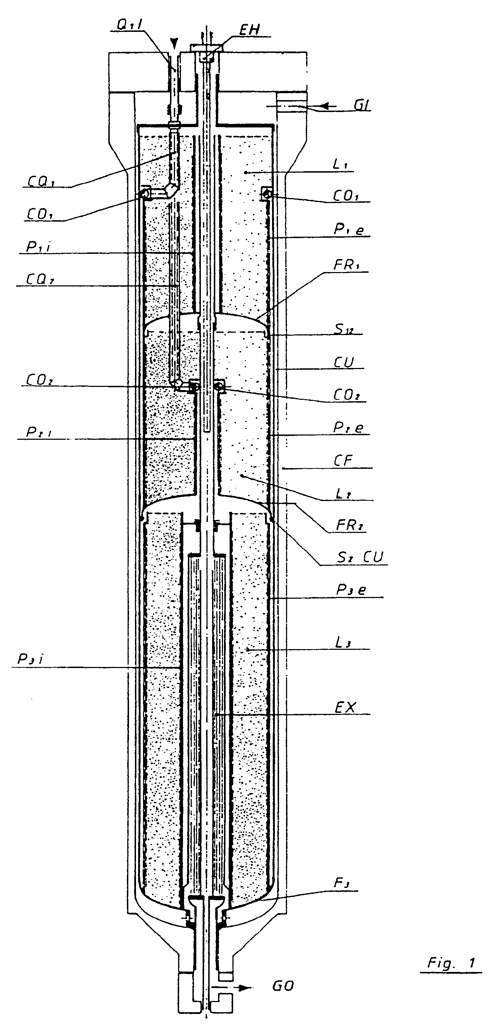

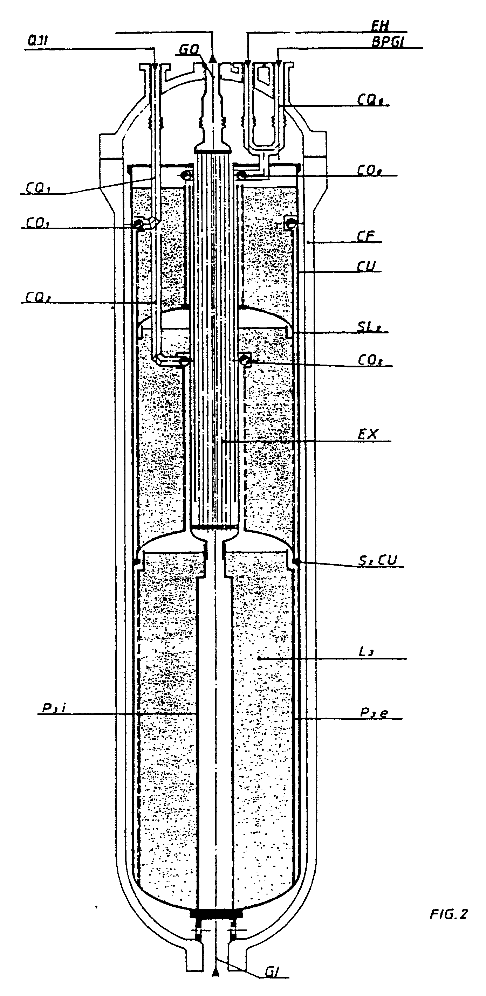

In Figures 1 and 2, CF indicates the external shell ; CU the cartridge ; L1, L2, L3 the three catalytic beds, each delimited by substantially perforated external walls P1e, P2e, P3e and internal walls P1i, P2i, P3i ; CQ1 and CQ2 the ducts feeding quench gas Q1 and Q2 to collectors CO1 and CO2 ; EX the gas/gas heat exchanger ; Q1I, Q2I and GI the inlets for quench gas, respectively synthesis gas ; CO1 and CO2 the collectors-distributors of quench gas ; S12 the support for bed L1 at the top of bed L2 and S2 CU the support for L2 on a ring of cartridge CU.

In Fig. 1 quench gas Q1 and Q2 enter from the top (inlet Q1I, while Q2I is hidden by Q1I) and through ducts CQ1, CQ2 arrive at collectors CO1 respectively CO2 situated on the outer periphery (corresponding to the top of P1i) of L1, respectively on the inner part (at the top of P2i) of L2. Quench gas Q1, therefore, flows outwardly, while second quench gas Q2 flows inwardly.

The basket of L1 rests on support S12 situated at the top of L2 and is therefore independent of cartridge CU, while said second bed L2 rests on packing ring S2 CU integral with the shell of CU ; in this way a seal is easily achieved between the second and third bed. In Fig. 1 the third bed L3 is run through by exchanger EX and the reacted gas leaves from the bottom GO. EH is an electric heater and BPGI is the by-pass for the gas entering the reactor.

In Fig. 2 quench gas Q1 and quench gas Q2 and the supports for the first and second beds are still the same as in Fig. 1, but synthesis gas GI enters from the bottom, the reacted gas GO leaves from the top, a third quench CQo injects cold gas into the annular space above the first catalytic bed L1, and the exchanger EX runs through the two catalytic beds L2 and L1.

Variations and modifications can be made regarding the embodiments illustrated without affecting either the purpose or the spirit of the invention.

## Claims

1. Reactor for exothermic heterogeneous catalytic synthesis under pressure, and more particularly for the catalytic synthesis of ammonia, methanol etc., consisting of an outer shell, of an internal cartridge, of several catalytic beds formed by baskets consisting of coaxial cylindrical walls enclosing a granular catalyst, said baskets comprising two baskets at the upper and lower ends and at least one intermediate basket, and of means to adjust and transfer reaction heat, characterized by the fact that :

— the cartridge-forming shell is substantially independent of the catalytic baskets ;

— at least a first end basket rests on an intermediate basket which in turn rests on a packing ring integral with said cartridge shell, the bottoms of said two baskets coupled together being inversely curved with respect to the curve at the bottom of the reactor ;

— in said first end bed a quench collector is placed between the basket's outer wall and the inside face of the cartridge shell ;

— in said intermediate bed a quench collector is placed inside the internal cylindrical wall forming the basket ; and

— a gas/gas heat exchanger is placed in a central position inside the cylindrical wall of at least the catalytic basket closest to the reactor outlet for hot reacted gas.

2. Reactor according to claim 1, characterized by the fact that said reactor comprises three beds, the upper bed has the quench collector on its outer periphery with centripetal flow of the quench gas, the intermediate bed resting on the packing ring and acting as support for said first upper basket has the quench collector inside its internal wall with centrifugal flow of the quench gas, the third bed has running through its centre the gas/gas exchanger, the synthesis gas and the quench gas being fed from the top while the reacted gas leaves from the bottom.

3. Reactor according to claim 1, characterized by the fact that the upper bed with quench collector on its outer periphery, as well as the intermediate basket with quench collector inside its internal wall are both run through the centre by a gas/gas exchanger, the synthesis gas being fed from the bottom, the quench gas being fed from the top, and with the reacted gas outlet placed at the top.

## Patentansprüche

1. Reaktor für eine exotherme, heterogene, katalytische Synthese unter Druck, und insbesondere für die katalytische Synthese von Ammoniak, Methanol usw., bestehend aus einem äußeren Mantel, aus einem inneren Einsatz, aus mehreren katalytischen Betten, die von Körben gebildet sind, die aus koaxialen, zylindrischen, einen körnigen Katalysator umschließenden Wänden bestehen, wobei die Körbe zwei Körbe am oberen und unteren Ende und wenigstens einen dazwischenliegenden Korb aufweisen, und aus Einrichtungen zum Einstellen und Übertragen von Reaktionswärme, dadurch gekennzeichnet, daß :

— die den Einsatz bildende Hülle von den katalytischen Körben im wesentlichen unabhängig ist ;

— wenigstens ein erster Endkorb auf einem dazwischenliegenden Korb ruht, der seinerseits auf einem mit der Einsatzhülle ein Stück bildenden Dichtungsring ruht, wobei die Böden der beiden zusammengekoppelten Körbe bezüglich der Krümmung am Boden des Reaktors umgekehrt gekrümmt sind ;

— in dem ersten Endbett zwischen der Außenwand des Korbs und der Innenfläche der Einsatzhülle ein Schnellkühlkollektor angeordnet ist ;

— in dem dazwischenliegenden Bett auf der Innenseite der inneren zylindrischen Wand, die den Korb bildet, ein Schnellkühlkollektor angeordnet ist ; und

— ein Gas/Gas Wärmeaustauscher in einer zentralen Position auf der Innenseite der zylindrischen Wand wenigstens des katalytischen Korbs angeordnet ist, der dem Reaktorauslaß für heißes Gas, das reagiert hat, am nächsten liegt.

2. Reaktor nach Anspruch 1, dadurch gekennzeichnet, daß der Reaktor drei Betten aufweist, das obere Bett bei zentripetalem Strom des Schnellkühlgases den Schnellkühlkollektor an seinem äußeren Umfang aufweist, das dazwischenliegende Bett, das auf dem Dichtungsring ruht und als Stütze für den ersten oberen Korb wirkt, bei zentrifugalem Strom des Schnellkühlgases den Schnellkühlkollektor innerhalb seiner inneren Wand hat und der Gas/Gas Austauscher sich durch das Zentrum des dritten Betts erstreckt, wobei das Synthesegas und das Schnellkühlgas von oben eingespeist werden, während das Gas, das reagiert hat, am Boden abströmt.

3. Reaktor nach Anspruch 1, dadurch gekennzeichnet, daß sich durch das Zentrum sowohl des oberen Betts mit Schnellkühlkollektor auf seinem äußeren Umfang wie auch des dazwischenliegenden Korbs mit Schnellkühlkollektor innerhalb seiner inneren Wand ein Gas/Gas Austauscher erstreckt, wobei das Synthesegas vom Boden und das Schnellkühlgas von oben eingespeist wird, wobei der Auslaß für das Gas, das reagiert hat, oben angeordnet ist.

## Revendications

1. Réacteur de synthèse catalytique hétérogène exothermique sous pression et, en particulier, de synthèse catalytique de l'ammoniac, du méthanol, etc., comprenant une enveloppe extérieure, une cartouche intérieure, plusieurs lits catalytiques formés de paniers comportant des parois cylindriques coaxiales enfermant un catalyseur sous forme granulaire, lesdits paniers comprenant deux paniers aux extrémités supérieure et inférieure et, au moins, un panier intermédiaire, et des moyens pour régler et transférer la chaleur de réaction, caractérisé en ce que :

— l'enveloppe formant cartouche est sensiblement indépendante des paniers catalytiques ;

— au moins un panier de la première extrémité repose sur un panier intermédiaire qui, de son côté, repose sur une garniture annulaire monobloc avec ladite enveloppe de cartouche, les fonds desdits deux paniers accouplés mutuellement étant incurvés en sens contraire par rapport à la courbe du fond du réacteur ;

— un collecteur de gaz de refroidissement est placé dans ledit lit de la première extrémité, entre la paroi extérieure du panier et la face interne de l'enveloppe de cartouche ;

— un collecteur de gaz de refroidissement est placé dans ledit lit intermédiaire, à l'intérieur de la paroi cylindrique intérieure formant le panier ; et

— qu'un échangeur gaz/gaz est placé en position centrale à l'intérieur de la paroi cylindrique d'au moins le panier catalytique le plus proche de la sortie des gaz de réaction chauds du réacteur.

2. Réacteur selon la revendication 1, caractérisé en ce que ledit réacteur comprend trois lits, le lit supérieur étant pourvu d'un collecteur de gaz de refroidissement sur sa périphérie extérieure, assurant un courant de gaz de refroidissement brutal centripète, le lit intermédiaire reposant sur la garniture annulaire et servant de support pour ledit premier panier supérieur ayant un collecteur de gaz de refroidissement à l'intérieur de sa paroi interne, assurant un courant centrifuge du gaz de refroidissement brutal, le troisième lit étant traversé à son centre par l'échangeur gaz/gaz, le gaz de synthèse et le gaz de refroidissement brutal arrivant par le haut tandis que le gaz de réaction sort par le bas.

3. Réacteur selon la revendication 1, caractérisé en ce que le lit supérieur pourvu d'un collecteur de gaz de refroidissement sur sa périphérie extérieure, ainsi que le panier intermédiaire pourvu d'un collecteur de gaz de refroidissement à l'intérieur de sa paroi interne sont tous deux traversés par un échangeur gaz/gaz, le gaz de synthèse étant alimenté par le fond, le gaz de refroidissement brutal étant alimenté par le haut, la sortie du gaz de réaction étant placée au sommet.

Fig. 1

FIG.2